# EUROPÄISCHE PATENTANMELDUNG

(11) **EP 1 889 923 A1**
(43) Veröffentlichungstag der Anmeldung: **20.02.2008**
(21) Anmeldenummer: 07014304.5
(22) Anmeldetag: 20.07.2007
(51) Int. Cl.: C12Q 1/68

(54) **Verfahren zur nicht radioaktiven Markierung von Ribonukleinsäuren in vivo**

(30) Priorität: 17.08.2006 DE 102006038686
(71) Anmelder: Forschungszentrum Karlsruhe GmbH, 76133 Karlsruhe (DE)
(72) Erfinder: Berensmeier, Sonja, Dr., 76131 Karlsruhe (DE); Schwartz, Thomas, Dr., 76131 Karlsruhe (DE); Stankiewicz, Nikolai, 76137 Karlsruhe (DE)
(74) Vertreter: Rückert, Friedrich

(57) **Zusammenfassung**

Verfahren zur nicht radioaktiven Markierung der im Verlauf der zellulären Transkription bei lebenden Organismen *(in vivo)* neu gebildeter Ribonukleinsäuren (RNA), unter Verwendung von markierten Nukleotiden und deren kovalenten Einbaus in mindestens eine dieser Ribonukleinsäure und Verwendung des Verfahrens zur Detektion als auch zur Separation von Ribonukleinsäuren aus einem Zellhomogenisat von lebenden Organismen.

## Beschreibung

Verfahren zur nicht radioaktiven Markierung der im Verlauf der zellulären Transkription bei lebenden Organismen (in vivo) neu gebildeter Ribonukleinsäuren (RNA), unter Verwendung von markierten Nukleotiden und deren kovalenten Einbaus in mindestens eine dieser Ribonukleinsäure.

Die isolierte Gesamt RNA besteht aus einem Gemisch aus ribosomaler RNA (rRNA), Transfer-RNA (tRNA), messenger-RNA (mRNA). Um die nur gering konzentrierte (1-4%) nichtstabile mRNA zu isolieren, wird bei Eukaryonten auf die PolyA-Verlängerung der mRNA selektiert, wobei jedoch andere RNAs, die adenosinreiche Sequenzen enthalten, durch das Verfahren mitisoliert werden. Bei Prokaryonten (Bakterien) besitzt die mRNA keinen stabilen PolyA-Anhang und kann somit nicht oder nur unter erheblichem Aufwand separiert werden. Die Reinigung beruht bei polyadenylierter RNA in aller Regel auf einer Hybridisierung an OligodT-Nukleotide von etwa 20 Basen Länge, die an einer Matrix gebunden sind. Die Matrix kann aus Cellulose, magnetischen Beads oder Latexkugeln bestehen. Um das Problem der fehlenden PolyA-Sequenz bei Bakterien zu umgehen, wurden verschiedene Strategien entwickelt, von denen jedoch keine universell einsetzbar war.

Wendisch et al. (2001) beschreibt ein Verfahren zur Reinigung von mRNA durch Polyadenylierung mit *E*. *coli* Polymerase I in rohem, mechanisch aufgeschlossenem Zellextrakt. Diese Methode konnte jedoch nur in wenigen Bakterienarten angewendet werden. Affymetrix Inc. (Rosenow et al. 2001) kann auch eine Anreicherung von *E*. *coli* mRNA durch eine Aneinanderreihung enzymatischer Schritte, die die 16S und 23S rRNA aus der Gesamt-RNA abbauen, erreichen. Dabei werden Reverse Transkriptase und spezifische Primer der 16S und 23S rRNA zur Synthese komplementärer DNA benutzt. Anschließend wird die rRNA durch RNase H entfernt, die spezifisch die RNA eines RNA-DNA-Hybrids erkennt.

Die verbleibende cDNA wird dann durch DNase I zerstört und die angereicherte mRNA wird gereinigt. Dieses Verfahren ist aufwendig und schwierig handhabbar und arbeitet nicht an lebenden Zellen (in vivo).

Der MICROExpress^{™} Bacterial mRNA Reinigungs-Kit (Firma Ambion), entfernt mit einem modifizierten Capture-Hybridisierungs-Ansatz die reichlich vorhandene 16S und 23S rRNA aus zuvor gereinigter Gesamt-RNA. Gereinigte Gesamt RNA wird dazu kurz in einem CaptureOligonukleotid-Mix in Hybridisierungspuffer inkubiert. Anschließend werden derivatisierte magnetische Partikel, die über Capture-Oligonukleotiden rRNA hybridisieren können, hinzu gegeben. Die magnetischen Partikel an denen die 16S und 23S rRNA gebunden sind, werden durch einen Magneten vom Gemisch abgetrennt und die verbleibende RNA wird durch eine Ethanolpräzipitation aufkonzentriert. Die Methode ist schnell und einfach, doch können nicht die tRNA, 5S rRNA und andere kleine RNAs entfernt werden und ebenso kann sie nicht bei allen Bakterienarten angewendet werden. Der Lösungsansatz eines Capture-Hybridisierungsverfahren wird in vielen Anwendungsgebieten wie z. B. der Reinigung von Antikörpern, Proteinen, DNA und eukaryontischer mRNA verwendet. Die Methode ist zur Isolierung eukaryontischer RNA gut etabliert. Auch für Prokaryonten konnten Bach et al. (1999) speziell die mRNA einer Protease mit bekannter Gensequenz aus *Bacillus cereus* mit dieser Technik reinigen. Verglichen mit herkömmlichen mRNA Reinigungsverfahren ist die magnetische Capture-Hybridisierungsmethode schnell und einfach, unterdrückt eine schnelle RNA-Degradation durch zelleigene und fremde RNasen. Weiterhin ist die isolierte mRNA (oder DNA) frei von jeglichen inhibierenden Komponenten, so dass sie direkt ohne weitere Behandlung für RT-PCR oder PCR eingesetzt werden kann. Neben diesen Vorteilen, überwiegen jedoch die folgenden Nachteile des Capture-Hybridisierungs-Verfahrens. Die mit dieser Methode isolierte mRNA ist nicht rein, sondern enthält diverse andere Ribonukleinsäuren, die mit einer späteren PCR-Analyse interferieren können. In einer, auf diese Weise isolierten Gesamt-mRNA, können demnach nur Expressionsanalysen spezifischer Gene durchgeführt werden. Auch erlaubt diese Methode keine zeitaufgelöste Analyse der Expression, da die mRNA unabhängig vom Zeitpunkt ihrer Expression isoliert wird.

Weitere Verfahren, die unter dem Stichwort des in vivo Labelings in der Literatur aufgeführt werden, sind Methoden der in situ-Hybridisierung. Hierbei werden die zu markierenden Zellen fixiert und permeabilisiert, so dass diese Arbeiten nicht im intakten, lebenden Organismus durchgeführt werden. Die Bindung der Markierung an die zelluläre RNA erfolgt außerdem nicht kovalent, sondern über eine Hybridisierungsreaktion.

Die Isolierung und Identifizierung von prokaryontischer Ribonukleinsäure (RNA) ist sehr wichtig, aber bei heutigem Stand der Technik sehr schwierig und aufwendig. Es besteht dringender Bedarf an einem einfachen und schnellen Verfahren des *in vivo* Labelings zur Detektion und Separation / Isolierung von prokaryontischer Ribonukleinsäure.

Das vorliegende erfindungsgemäße Verfahren gemäß dem Anspruch 1, Verfahren zur nicht radioaktiven Markierung der im Verlauf der zellulären **Transkription** bei lebenden Organismen neu gebildeter Ribonukleinsäuren (RNA), unter Verwendung von markierten Nucleotiden und deren kovalenten Einbaus in mindestens eine dieser Ribonukleinsäure, löst diese Aufgabe.

Das erfindungsgemäße Verfahren ist universell verwendbar und nicht auf einzelne Gene oder Organismen beschränkt. Da es in der intakten Zelle/ Organismen (in vivo) durchgeführt wird, liefert es Ergebnisse aus lebenden Zellen/ Organismen, die nicht zuvor fixiert oder permeabilisiert wurden. Die kovalent eingefügte Markierung, auch Label genannt, kann im Gegensatz zum radioaktiven Label sowohl zur Separation/ Isolierung als auch zur Detektion eingesetzt werden. Dieses Verfahren erleichtert bisher bestehende Arbeitsabläufe maßgeblich, da es die integrierte Durchführung der Prozessschritte Markierung und Reinigung von RNA ermöglicht und nicht den Einsatz zweier separater Verfahrensschritte erfordert. Das vorliegende Verfahren erlaubt das kovalente Labeling expremierter RNA in lebenden prokaryontischen Organismen, insbesondere in Bakterien. Alle bisher für Bakterien beschriebenen Methoden nutzen lediglich radioaktive markierte Moleküle oder arbeiten *in vitro.*

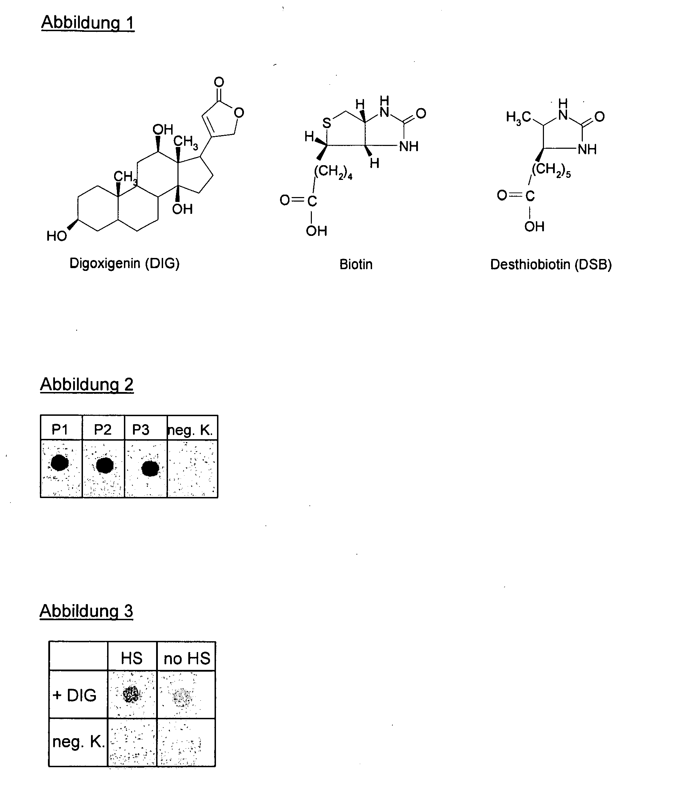

Das vorliegende, nicht radioaktive, in vivo Labeling ermöglicht die Durchführung von Expressionsanalysen und kann bei Mischpopulationen unbekannter Zusammensetzung und unbekannter molekularer Regulation von Genen durchgeführt werden. Das vorliegende *in vivo* Labeln ermöglicht die Identifizierung der bakteriellen RNA von aktiv expremierten Genen, zur anschließenden schnellen und einfachen Reinigung und Separation, zum Beispiel mittels magnetischer Träger, so genannter Magnetbeads, oder Affinitätschromatographie. Die jeweiligen Oberflächen (Matrix), sind so oberflächenmodifiziert, dass DIG, Biotin, Desthiobiotin (Abbildung 1) und anders gelabelte RNA an Affinitätsliganden spezifisch gebunden und somit zielgerichtet einem Zellhomogenisat entzogen wird. Die separierte/ isolierte RNA steht anschließend für Genexpressionsstudien zur Verfügung. Das vorliegende erfindungsgemäße Verfahren hat den Vorteil, dass ein in vivo Labeln der RNA lebender Bakterien voraus läuft und somit neben einer vereinfachten Reinigung auch zugleich eine vereinfachte Quantifizierung der de novo Genexpression ermöglicht wird. Unter einem nicht-radioaktivem *in vivo* Labeling versteht man hierbei die Aufnahme z.B. Hapten-modifizierter Nukleotide durch Bakterien und deren Einbau in die neu synthetisierte RNA.

Eine Einheit aus einer Base (Purin, Pyrimidin) und einem Zucker (Ribose) wird als Nukleosid bezeichnet. Die vier in RNA vorkommenden Neukleosideinheiten heißen Adenosin, Guanosin, Cytidin und Uridin. In allen Fällen ist das N-9-Atom eines Purins beziehungsweise des N-1-Atom eines Pyrimidins mit dem C-1'-Atom eines Zuckers verknüpft. Ein Nukleotid ist ein Nukleosid, das über eine Esterbindung mit einer oder mehreren Phosphatgruppen verbunden ist. Die markierten Nukleotide sind entweder ein Pyrimidinderivat von Uracil oder Cytosin, oder ein Purinderivat von Adenin oder Guanin, bei diese Nukleotide ist das Nukleosid mit einem Hapten markiert, welches vorzugsweise gebundenes Digoxigenin und/oder Biotin und/oder Desthiobiotin ist oder ein anderes diese Funktion erfüllende spezifische Markerverbindung, wie Antikörper oder synthetische Signalproteine. Diese Markerverbindungen können auch Fluoreszenz markiert sein. Das vorliegende erfindungsgemäße Verfahren hat keine Gemeinsamkeiten mit Verfahren, die vorher isolierte Nukleinsäuremoleküle zur Markierung einsetzen, also in vitro arbeiten (Odom et al. 1988). Zu solchen Methoden zählen all diejenigen, die isolierte RNA kovalent enzymatisch markieren oder markierte RNA durch Endlabeling synthetisieren (Schmitz et al. 1991; Gauthier et al. 2003; *DIG Oligonukleotide 3'-End Labeling Kit, 2nd generation,* Artikelnr. 03353575910 (Firma Roche Diagnostics GmbH). Ein in vivo Labeling bakterieller RNA wurde bisher ausschließlich für radioaktive Label beschrieben (Baracchini et al. 1987). Das vorliegende Verfahren jedoch setzt keinerlei radioaktiv markierte Nukleotide (dNTP's) ein. Alle für Prokaryonten bisher beschriebenen und bestehenden Verfahren basieren entweder auf dem Einbau radioaktiv markierter Nukleotide oder der Fütterung radioaktiver Substrate (Baracchini et al. 1987). Ein von den Bakterien selbst vollzogener Einbau nicht-radioaktiv markierter Nukleotide wurde bisher nicht beschrieben. Des Weiteren kann eine radioaktive bzw. eine Fluoreszenz-Markierung lediglich zur Detektion, nicht aber als Zielstruktur für eine affinitätschromatographische Aufreinigung genutzt werden. Somit ist es mit Separationstechniken nicht möglich, mit bisher bekannten Techniken gelabelte RNA-Molekülen von nicht gelabelten RNA-Molekülen zu unterscheiden und weiterführend zu analysieren. Ein weiterer Vorteil des vorliegenden Verfahrens ist also nicht nur im Einsatz eines Labels zur Detektion, sondern darin zu sehen, dass dieses Label ebenso zur Separation/ Isolierung herangezogen werden kann, was durch eine radioaktive- bzw., Fluoreszenz-Markierung nicht möglich ist.

Digoxigenin (DIG) ist ein steroides Hapten, welches von spezifischen Antikörpern erkannt und gebunden wird. Geeignete DIG-Antikörper die an eine Matrix gebunden werden können sind kommerziell erhältlich. Der Komplex zwischen dem Vitamin Biotin und dem Eiweiß Glykoprotein Avidin (oder dem verwandten Bakterienprotein Streptavidin) ist die bisher stärkste bekannte Ligand-Protein-Interaktion (*K*ₐ ~ 10¹⁵M⁻¹). Für die geplante Zielsetzung ist diese starke Bindung eher hinderlich, da extreme denaturierende Bedingungen zum Lösen des Komplexes und somit zur Abtrennung des RNA von der Matrix, wie zum Beispiel Magnetbeads, nötig wären und zur Zerstörung der RNA führen würden. Daher ist es sinnvoll, für die Funktionalisierung der Matrix, auf (Strept)avidin Varianten zurückzugreifen, die stark, aber reversibel Biotin binden. Morag et al. (1996) konnte (Strept)avidin durch Nitrieren des Tyrosins in der Biotin-Bindungsstelle chemisch modifizieren, so dass eine Interaktion mit Biotin unter relativ milden Bedingungen gelöst werden kann. Eine weitere Methode des Nukleotid markierens könnte mit Desthiobiotin erfolgen. Desthiobiotin (DSB) ist ein Analogon des Biotin und wird weniger stark von biotin-bindenden Proteinen gebunden und kann einfach kompetitiv durch Biotin entfernt werden. Hirsch *et al*. (2002) synthetisierten aminreaktive Desthiobiotin Derivate zum Labeln von Proteinen und zeigten, dass sie wie ihre biotinylierten Gegenstücke an Streptavidin und anderen biotin-bindenden Affinitätsmaterialen binden sowie von Biotin Antikörpern erkannt werden. Die Firma Molecular Probes Inc. (U.S.A.) bietet einen DSB-X Labeling Kit an, bei dem DSB-X mit primären Aminen der Proteine oder modifizierten Nukleinsäuren unter Bildung einer Aminbindung reagiert.

Derzeit gibt es aufgrund der fehlenden PolyA-Verlängerung der mRNA kein Verfahren de novo gebildete und Markierungsspezifische mRNA/rRNA direkt aus Bakterien zu isolieren. Das hier vorliegende in vivo Labeln bakterieller RNA ermöglicht zum einem das direkte und schnelle Isolieren mittels funktionalisierte Oberflächen (Matrix) und zum anderen genauere Expressionsanalysen. Es konnte gezeigt werden, dass Bakterien (Grampositive und Gram-negative) ein markiertes Nukleotid (DIG-dUTP) unabhängig von Wachstumsphase und Zelldichte aufnehmen und in die RNA einbauen.
DIG-UTP wurde in Teile der Gesamt-RNA von Gram negativen (Pseu*domonas aeruginosa*) und Gram-positiven Bakterien (Enterococcus *faecium*) eingebaut. Mittels Northern dot Blot Verfahren wurde dies gezeigt. Durch Wechsel der Temperaturverhältnisse des Mediums bzw. bei Anwesenheit von zweiwertigen Ionen wurde der Einbau zusätzlich erhöht. Ziel ist allerdings das Verfahren einfach zu gestalten, d.h. die Inkubation in oligotrophen Verhältnisse reicht für die weitere Prozessierung der markierten RNA aus. Gezielt konnte die markierte de novo RNA mittels DIGspezifischen Antikörpers von der Gesamt RNA abgetrennt werden. Die Separation erfolgte über Magnetic bead Technologie. Die isolierte Gesamt-RNA aus den Inkubationsansätzen wurde quantifiziert und die Markierungsraten im Blot-Verfahren und Antikörper-vermittelter Chemilumineszenz erfasst. Die Bindungseffizienz der markierten RNA-Moleküle der Referenzbakterien wurde bereits mit vorbereiteten Beads überprüft und nach Abtrennung der Beads von Gesamtextrakt eine RNA-spezifische Quantifizierung durchgeführt. Im Blot- und Chemilumineszenzverfahren wurde die Markierungseffizienz aus dem RNA Gesamtextrakt mit der Effizienz der RNA-Markierung nach Beadbehandlung densitometrisch verglichen. Da ribosomale RNA (rRNA)- Sequenzen vornehmlich zur taxonomischen Charakterisierung von bakteriellen Biozönosen benutzt werden, während die Boten-RNA (mRNA) die Funktionalität der aktiven Gene bestimmt, ist es möglich mittels kommerziell erhältlicher Separationsverfahren die rRNA von der mRNA abzutrennen. Durch parallele Untersuchungen werden beide RNA-Typen auf ihre Markierungseffizienz durch den Einbau markierter Nukleotide untersucht. Durch Sequenzierung der separierten rRNA können zukünftig auf diesem Wege unbekannte oder nicht kultivierbare Bakterien aus der Umwelt typisiert werden.

Für eukaryontische Zellen kann der Austausch radioaktiver- gegen nicht-radioaktive Markierungen auf Ebene eines *in vivo* Labelings von Nukleinsäuren ab dem Jahr 1990 beobachtet werden (Aglietta et al. 1991). Als Ersatz für eine radioaktive Markierung lebender eukaryontischer Zellen werden zumeist Fluoreszenzfarbstoffe eingesetzt. Das vorliegende Verfahren bezieht sich jedoch ausschließlich auf Organismen aus dem biologischen Reich der Prokaryonten. Das erfindungsgemäße Verfahren schließt die Lücke zwischen struktureller und funktioneller Vielfalt zellulärer (pro- und eukaryontischer) Verbände, da verschiedene Genexpressionen bestimmter Organismen unter unterschiedlichen Bedingungen gezeigt werden können. Genexpressionsstudien auf der Basis von Mikroarrays setzen häufig die Synthese von messenger RNA komplementärer cDNA voraus, die wiederum auf der Hybridisierung bekannter Nukleotidsequenzen basiert. Eukaryontische mRNA ist, im Gegensatz zu der prokaryontischen mRNA, am 3'-Terminus stabil polyadenyliert und ermöglicht dadurch eine Reinigung über die Hybridisierung mit Oligo(dT)-Nukleotiden, die an einer Matrix befestigt sind. Um jedoch bakterielle RNA erfassen und damit eine quantifizierte Aussage über die de *novo* Genexpression machen zu können, ist ein direktes in vivo Labeln der Gesamt-RNA notwendig. Weiterführend wird aufgrund des verstärkten Einbaus eines *in vivo* Labels in die spezifisch induzierten mRNAs die Funktionalität der Gene bei Organismen in ihrer natürlichen Umgebung erkannt. Die Identifizierung der neusynthetisierten mRNA bzw. rRNA Moleküle erfolgt durch die gezielte Separation / Isolation über das eingeführte Label-Molekül.

Mit den Informationen, wie z. B. bakterielle Funktionen *in situ* im natürlichen Habitat gesteuert werden, wird die Voraussetzung geschaffen, gezielt bakterielle Prozesse in z. B. technischen Abläufen (Aufbereitungsanlagen, Kläranlagen) zu steuern. Im Gegensatz zu Eukaryonten finden Prokaryonten eine breite industrielle Anwendung und das hier vorliegende Verfahren kann die Erforschung der Prokaryonten stark erleichtern.

### Beispiele:

Nachfolgend wird ein Beispiel des erfindungsgemäßen Verfahrens tabellarisch dargestellt.

Protokoll des *in vivo* Labelings:
1. Kulturwachstum bei 37°C bis zu einer OD⁶⁰⁰ zwischen 0,05 und 2
2. auf Eis aliquotieren in 1 ml Fraktionen
3. Zentrifugation für 5 Minuten bei 10000 upm; Überstand verwerfen
4. Pellets in 500 µl VE-H₂O resuspendieren
5. 30 min auf Eis inkubieren
6. Zentrifugation 5 Minuten bei 10000 upm; Überstand verwerfen
7. Pellets in 100 µl 0,9% NaC1-Lösung oder PBS resuspendieren
8. Aliquots mit 1 nmol Digoxigenin versetzen
9. 10 Minuten auf Eis inkubieren
10. Optional zur Erhöhung der Ausbeute: Schock Behandlung [z.B. Hitze Schock]
11. 900 µl SOC-Medium zugeben
12. 1,5 Stunden bei 37°C im Schüttler inkubieren
13. 5 Minuten bei 10000 upm zentrifugieren
14. Pellets 2 Mal in 200 µl PBS(5 Minuten bei 10000 upm zentrifugieren)

Mit diesem Protokoll können Bakterienzellen unabhängig von ihrer jeweiligen Wachstumsphase, d.h. unabhängig ihrer optischen Dichte gelabelt werden. Abbildung 2 *in vivo* gelabelte *Pseudomonas aeruginosa* RNA zeigt den Nachweis der gelabelten RNA im Northern dot Blot Verfahren. Der Nachweis erfolgte mittels Chemilumineszenz durch ein spezifisches Anti-DIG-Antikörper-Alkine-Phospatase Konjugat. Zellen optional mit Hite Schock behandelt. [P1 - P3: parallele Labeling Ansätze; neg. K.: Negativ Kontrolle ohne DIG Zugabe; 1 µg RNA aufgetragen; 15 min Belichtungszeit].

Bei dem oben dargestellten Ergebnis wurde der optionale Hitzeschock-Schritt durchgeführt, um die Ausbeute des Labelings zu erhöhen. Die Abbildung 3 in vivo gelabelte *Pseudomonas aeruginosa* RNA, zeigt den Nachweis, dass ein Labeling auch ohne einen Hitze Schock möglich ist, dieser jedoch die Markierungsrate stark erhöht.

[HS: mit Hitze Schock; no HS: ohne Hitze Schock; + DIG: mit DIG gelabelt; neg. K.: Negativ Kontrolle ohne DIG Zugabe; 1 µg RNA aufgetragen; 15 min Belichtungszeit für Chemilumineszenzdetektion].

### Literatur:

Aglietta M., Monzeglio C., Sanavio F. et al. (1991). "In vivo effect of granulocyte-macrophage colony-stimulating factor on the kinetics of human acute myeloid leukemia cells." Leukemia. 5(11): 979-984.
Bach, H. J., A. Hartmann, J. T. Trevors and J. C. Munch (1999). "Magnetic capture-hybridization method for purification and probing of mRNA for neutral protease of Bacillus cereus." J. Microbiol. Methods 37: 187-192.
Baracchini E., Bremer H. (1987). "Determination of synthesis rate and lifetime of bacterial mRNAs." Anal Biochem. 167 (2) : 245-260.
Hirsch, J. D., L. Eslamizar, B. J. Filanoski, N. Malekzadeh, R. P. Haugland, J. M. Beechem and R. P. Haugland (2002). "Easily reversible desthiobiotin binding to streptavidin, avidin, and other biotin-binding proteins: uses for protein labeling, detection, and isolation." Anal. Biochem. 308: 343-357.
Morag, E., E. A. Bayer and M. Wilchek (1996). "Reversibility of biotin-binding by selective modification of tyrosine in avidin." Biochem. J. 316: 193-199.
Odom O. W., Deng H. Y., Hardesty B. (1988). "Fluorescence labeling and isolation of labeled RNA and ribosomal proteins." Methods Enzymol. 164: 174-187.
Rosenow, C., R. M. Saxena, M. Durst and T. R. Gingeras (2001). "Prokaryontic RNA preparation methods useful for high density array analysis: comparison of two approaches." Nucleic Acids Res. 29: No. 22e112.
Schmitz G. G., Walter T., Seibl R., Kessler C. (1991). "Nonradioactive labeling of oligonucleotides in vitro with the hapten DIGoxigenin by tailing with terminal transferase." Anal Biochem. 192(1): 222-231.
Wendisch, V. F., D. P. Zimmer, A. Khodursky, B. Peter, N. Cozzarelli and S. Kustu (2001). "Isolation of Escherichia coli mRNA and comparison of expression using mRNA and total RNA and DNA microarrays." Anal. Biochem. 290: 205-213.

## Patentansprüche

1. Verfahren zur nicht radioaktiven Markierung der im Verlauf der zellulären **Transkription** bei lebenden Organismen neu gebildeter Ribonukleinsäuren (RNA), unter Verwendung von markierten Nukleotiden und deren kovalenten Einbaus in mindestens eine dieser Ribonukleinsäure.

2. Verfahren nach Anspruch 1 **dadurch gekennzeichnet, dass** die lebenden Organismen Prokaryonten sind.

3. Verfahren nach Anspruch 1 **dadurch gekennzeichnet**, das die Ribonukleinsäure ribosomale RNA (rRNA) und/oder Transfer-RNA (tRNA) und/oder messenger-RNA (mRNA) ist.

4. Verfahren nach Anspruch 1 **dadurch gekennzeichnet, dass** die markierten Nukleotide entweder ein Pyrimidinderivat von Uracil oder Cytosin, oder ein Purinderivat von Adenin oder Guanin enthalten.

5. Verfahren nach Ansprüchen 1 bis 4 **dadurch gekennzeichnet, dass** die markierten Nukleotide mit einem Hapten zur Markierung modifizierte Nukleotide sind.

6. Verfahren nach Anspruch 1 bis 5 **dadurch gekennzeichnet, dass** das Hapten ein, an das Nukleotid kovalent gebundenes Digoxigenin und/oder Biotin und/oder Desthiobiotin ist.

7. Verwendung des Verfahrens nach Anspruch 1 sowohl zur Detektion als auch zur Separation von Ribonukleinsäuren in und/oder aus einem Zellhomogenisat von lebenden Organismen gemäß Anspruch 1.

8. Verwendung nach Anspruch 7 **dadurch gekennzeichnet**, das die Detektion und/oder Separation durch einen gegen ein Hapten, gemäß Anspruch 6, spezifischen Affinitätsliganden erfolgt, indem das Hapten spezifisch durch den Affinitätsliganden gebunden wird.

9. Verwendung nach Anspruch 7 und 8 **dadurch gekennzeichnet**, das der Affinitätsligand auf einer Matrix gebunden ist.
